# EUROPEAN PATENT SPECIFICATION

(11) **EP 3 763 402 B1**
(45) Date of publication and mention of the grant of the patent: **30.10.2024**
(21) Application number: 19185814.1
(22) Date of filing: 11.07.2019
(51) Int. Cl.: A61M 5/00, A61M 5/142, A61F 13/02

(54) **A JACKET FOR WATERTIGHT SEALING A MEDICAL DEVICE**
MANTEL ZUM WASSERDICHTEN VERSCHLIESSEN EINER MEDIZINISCHEN VORRICHTUNG
GAINE POUR LE SCELLEMENT ÉTANCHE D'UN DISPOSITIF MÉDICAL

(43) Date of publication of application: 13.01.2021
(73) Proprietor: Roche Diabetes Care GmbH, 68305 Mannheim (DE); F. Hoffmann-La Roche AG, 4070 Basel (CH)
(72) Inventor: VON CAMPENHAUSEN, Harald, 68305 Mannheim (DE)
(74) Representative: Freiherr von Campenhausen, Harald

(56) References cited:
- WO-A1-99/02110
- WO-A2-2007/092543
- GB-A- 2 460 394
- JP-A- 2018 138 116

## Description

### Technical Field

The invention relates to a jacket for watertight sealing a medical device and to a method for watertight sealing a medical device. The device and the method according to the present invention may mainly be used for encasing medical devices which are worn on extremities of a user or a patient such as on an arm of the user or the patient. The device may be configured for providing a protection for the medical device, specifically against moisture. Thus, the invention may specifically be applied while taking a shower or a bath and during swimming. Other applications are generally feasible.

### Background art

Delivering medicine to a user, specifically insulin delivery, plays an important role in the prevention and treatment of diseases, in particular in the treatment of diabetes mellitus. Besides using injection pens or syringes, insulin delivery may specifically be performed by using insulin pumps. In particular, a user is generally required to wear the insulin pump on his or her body at all times. Common pumps for delivering medicine, such as for example insulin, comprise a plurality of medicine reservoirs. As an example, fluid delivery devices are disclosed in WO2011/046950 A1. The fluid delivery device comprises a housing having a fluid reservoir. A needle is in fluid communication with the fluid reservoir in an engaged position and out of fluid communication with the fluid reservoir in armed and storage positions. A proximal end of a biasing member is coupled to the housing and a distal end of the biasing member is configured to deliver a force to the fluid reservoir. A piston member extends through the biasing member and is coupled to the distal end of the biasing member. The piston member is fixed with respect to the housing in a locked position such that the biasing member does not deliver the force to the fluid reservoir and the piston member is moveable with respect to the housing in a released position such that the biasing member delivers the force to the fluid reservoir. Transitioning the needle from the storage position to the armed position transitions the piston from the locked position to the released position.

Further, in the field of medical technology and diagnostics, a large number of devices and methods for determining a presence and/or a concentration of one or more analytes in samples, specifically fluid samples, such as body fluids, and/or for determining at least one parameter of a sample are known. For performing fast and simple measurements, several types of test elements are known, which mainly are based on the use of one or more test chemicals, i.e. on the use of one or more chemical substances, one or more chemical compounds or one or more chemical mixtures, adapted for performing a detection reaction for detecting the analyte or determining the parameter. The test chemical often is also referred to as a test substance, a test reagent, a test chemistry or as a detector substance. In addition to so-called spot measurements, in which a sample of a body fluid is taken from a user in a targeted fashion and examined with respect to the analyte concentration, continuous measurements are increasingly becoming established. Thus, in the recent past, continuous measuring of glucose in the interstitial tissue (also referred to as continuous monitoring, CM) for example has been established as another important method for managing, monitoring and controlling a diabetes state. In the process, the active sensor region is applied directly to the measurement site, which is generally arranged in the interstitial tissue, and, for example, converts glucose into electrical charge by using an enzyme (e.g. glucose oxidase, GOD), which charge is related to the glucose concentration and can be used as a measurement variable. Examples of such transcutaneous measurement systems are described in US 6,360,888 B1 or in US 2008/0242962 A1. Hence, current continuous monitoring systems typically are transcutaneous systems or subcutaneous systems, wherein both expressions, in the following, will be used equivalently. This means that the actual sensor or at least a measuring portion of the sensor is arranged under the skin of the user.

Commonly, medical pumps and devices determining a presence and/or a concentration of one or more analytes in samples comprise electrical components. Further, these devices may require energy sources such as batteries or accumulators. Further, such devices may be configured for attachment to a skin site of the user or the patient. As a consequence, measures have to be taken to protect these devices from an ambient environment and in particular from contact to humidity and liquids such as water.

US 5 817 038 A describes a system for supporting and protecting wounds, incisions, transdermal procedural sites, and associated medical equipment from moisture and other contaminants. The invention is an assembly of a waterproof outer cover, at least one water absorbent dam, and an equipment support sleeve deployable around a limb or extremity. The waterproof outer cover is a limp tube or sleeve with at least one waterproof closure providing an annular seal around a user's extremity. The water absorbent dam is detachably deployed just inside the seal around the extremity to absorb any moisture which breaches the seal during water exposure and to catch any retained moisture as the waterproof sleeve is removed. The support sleeve secures equipment to the extremity within the waterproof cover. The mounting of this system is cumbersome and cannot be readily accomplished by the user himself as various straps need to be secured.

US 7 658 719 A1 describes a bandage bag. The bandage bag provides the dual functions of holding a medical apparatus to a patient for daily wear and of serving as a waterproof bandage, especially for a catheter and its insertion site. The bandage bag comprises a bag and two adhesive layers. The bag opens along a first edge. One adhesive layer is placed along the other three edges on the anterior side of the bag. The second adhesive layer is placed along the first edge on the posterior side of the bag. The bag is attached to a patient's arm near the insertion site using the second adhesive layer. The catheter tubing and port is placed in the bag. The bag is then folded over so the first adhesive layer on the anterior side faces the arm and covers the insertion site, forming a waterproof bandage for the insertion site and the medical apparatus. This solution cannot be used for medical devices attached to the skin because the device needs to be inserted into the bag first and after bagging the device cannot be directly attached to the skin as it would be required, e.g. for transcutaneous devices such as infusion pumps, analyte measurement devices such as glucose measurement devices or any other medical device that requires direct attachment to the skin.

JP 2018/138116 A1 discloses a waterproof cover which is cylindrical, has waterproofness and flexibility, and openings through which an arm or leg is made to pass on both ends. At least one face of a pair of independent foam sponge tapes is on a slice face. The pair of independent foam sponge tapes is attached to each opening so that the slice face can be brought into watertight close contact with the arm or leg that is made to pass through each opening. A pair of surface zipper bands can fix the respective independent foam sponge tapes in such a state that the respective independent foam sponge tapes are brought into watertight close contact with the arm or leg that is made to pass through each opening and wound.

Despite the advantages of state of the art apparatuses for protecting a medical device from an ambient environment, several technical challenges remain. Specifically, a mounting of the apparatuses to the extremity of the user or the patient and an encasing of the medical device may be cumbersome. Further, the patient or the user may require additional help from another person to mount the apparatus.

### Problem to be solved

It is therefore an objective of the present invention to provide a jacket for watertight sealing a medical device and preferably for a medical device attached to the skin and a method for watertight sealing a medical device which at least partially avoid the shortcomings of known devices and methods of this kind and which at least partially address the above-mentioned challenges. Specifically, devices and methods shall be disclosed which allow for simple handling processes by a user or a patient.

### Summary

This problem is addressed by a jacket for watertight sealing a medical device and a method for watertight sealing a medical device with the features of the independent claims. Advantageous embodiments which might be realized in an isolated fashion or in any arbitrary combinations are listed in the dependent claims and throughout the specification.

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

The terms "patient" and "user" as used herein are broad terms and are to be given their ordinary and customary meaning to a person of ordinary skill in the art and are not to be limited to a special or customized meaning. The terms specifically may refer, without limitation, to a human being or an animal, independent from the fact that the human being or animal, respectively, may be in a healthy condition or may suffer from one or more diseases. As an example, the patient or the user may be a human being or an animal suffering from diabetes. However, additionally or alternatively, the invention may be applied to other types of users or patients or diseases.

In a first aspect of the present invention, a jacket for watertight sealing a medical device is disclosed. The jacket comprises at least one flexible envelope. The flexible envelope comprises at least one first side for facing the skin of a patient and at least one opposing second side. The first side comprises:
- at least one first sealing lip, wherein the first sealing lip is configured to surround the medical device; and
- at least one second sealing lip, wherein the second sealing lip is configured to surround the medical device, wherein the second sealing lip is spaced apart from the first sealing lip.

The flexible envelope further comprises at least one envelope contacting sealing end. The skin-facing first side of the envelope contacting sealing end is fixedly connectable to the opposing second side of the flexible envelope when wrapping the flexible envelope around an extremity of a user. Further, the second sealing lip surrounds the first sealing lip.

The term "jacket" as used herein is a broad term and is to be given its ordinary and customary meaning to a person of ordinary skill in the art and is not to be limited to a special or customized meaning. The term specifically may refer, without limitation, to an arbitrary object which may be placed around at least a part of another element. Specifically, the jacket according to the invention may be configured to be laid over or to be wrapped around the other object and in particular around the extremity. Thus, the jacket may serve as a covering, specifically as a protective covering shielding the part of the other element from an outer environment. The jacket may be configured to be mounted on the other element and to cover at least one surface of the other element. Specifically, the jacket may be configured to be in direct contact with the other element. Thus, the jacket may also be referred to as a cover, a covering or a coat.

The term "sealing" as used herein is a broad term and is to be given its ordinary and customary meaning to a person of ordinary skill in the art and is not to be limited to a special or customized meaning. The term specifically may refer, without limitation, to an object or a process of concealing at least one opening and/or of fully or partially surrounding one or more objects to be sealed off from environmental influences such as moisture, dust and/or dirt. The term "watertight sealing" as used herein is a broad term and is to be given its ordinary and customary meaning to a person of ordinary skill in the art and is not to be limited to a special or customized meaning. The term specifically may refer, without limitation, to an object or to a process of concealing at least one opening and/or of fully or partially surrounding one or more objects to be sealed off from humidity and/or water such that a penetration of water and/or humidity into the opening and/or into the object is prevented or at least reduced to a large extent. Preferably, the sealing according to the invention is a sealing which prevents water from contacting the medical device during use of the jacket if the water source is splash water, rain, a shower, a bath, swimming in a depth of up to 3 meter water depth, or up to 1 meter water depth, preferably for a time of up to 5 min, up to 10 min, up to 20 min or up to 30 minutes.

The term "medical device" as used herein is a broad term and is to be given its ordinary and customary meaning to a person of ordinary skill in the art and is not to be limited to a special or customized meaning. The term specifically may refer, without limitation, to an arbitrary device configured for performing at least one therapeutic purpose. The medical device specifically may comprise one component or an assembly of two or more components capable of interacting with each other, such as in order to perform one or more therapeutic purposes, such as in order to perform the medical procedure. The medical device generally may also be or may comprise at least one of a medical system or a medical kit.

The medical device generally may be used for delivering at least one medication such as a drug and/or a therapeutic agent to a user. Thus, the medical device may be used as part of one or more medical treatments. Thus, the medical device may specifically be a medication device for delivering at least one medication to a user. The medical device for delivering at least one medication to a user may exemplarily be selected from the group consisting of a medical pump, specifically an insulin pump; a remote control unit of a medical pump. However, also other embodiments of the medical device may be feasible.

Further, the medical device may be a medical device for detecting at least one analyte in a body fluid. As further used herein, the term "detecting" refers to a process of determining a presence and/or a quantity and/or a concentration of at least one analyte. Thus, the detection may be or may comprise a qualitative detection, simply determining the presence of the at least one analyte or the absence of the at least one analyte, and/or may be or may comprise a quantitative detection, which determines the quantity and/or the concentration of the at least one analyte. As a result of the detection, at least one signal may be produced which characterizes an outcome of the detection, such as at least one measurement signal.

Specifically, the medical device may preferably be a medical device which is adapted to be worn on or attached to the skin of a user or patient, in particular a transcutaneous medical device. As used herein, the term "transcutaneous medical device" refers to a medical device which is adapted to be worn on the skin and a part of the medical device is at least partly arranged within a body tissue, preferably inserted transcutaneously into the skin of the patient or the user and more preferably the inserted part is in physical contact with the remaining part of the medical device worn on the skin. The part which is adapted to be inserted transcutaneously into the skin may be also referred to as "transcutaneously positioned part" or "transcutaneously positionable part". For example, the medical device may be worn on the skin of a patient or user and comprises a transcutaneously insertable sensor or a needle or cannula. Suitable medical devices comprise analyte sensors such as glucose sensors including continuous glucose measurement devices such as the Dexcom G5 or G6 Continuous Glucose Monitoring (CGM) System or the Abbott Freestyle Libre Glucose Monitoring System. Also included are skin attachable drug delivery pump devices such as insulin pumps the deliver the drug through a transcutaneously placeable fluid infusing cannula or needle such as Insulet's Omnipod Insulin Management System patch pump or the Roche Accu-Chek Solo Micropump System. Moreover, also encompassed by the term "transcutaneous medical device" is a medical device where the transcutaneously inserted part of the medical device can be fully implanted into or under the skin and is able to wirelessly communicate with the part of the medical device worn on the skin such as in the case of the Senseonics Eversense Eversense Continuous Glucose Monitoring System. The transcutaneous medical device of the present invention generally may be dimensioned such that a transcutaneous insertion is feasible, such as by providing a width in a direction perpendicular to an insertion direction of no more than 5 mm, preferably of no more than 2 mm, more preferably of no more than 1.5 mm. The medical device may have a length of less than 100 mm, or less than 50 mm, such as a length of 30 mm or less, e.g. a length of 5 mm to 30 mm. The medical device may have a width of less than 100 mm, or less than 50 mm, such as a length of 30 mm or less, e.g. a width of 5 mm to 30 mm. The medical device may have a height of less than 40 mm, or less than 30 mm, such as a height of 20 mm or less, e.g. a height of 5 mm to 20 mm. It shall be noted, however, that other dimensions are feasible. In order to further render the medical device to be usable as a transcutaneous medical device, the medical device may fully or partially provide a biocompatible surface, i.e. a surface which, at least during durations of use, do not have any detrimental effects on the user, the patient or the body tissue.

The term "envelope" as used herein is a broad term and is to be given its ordinary and customary meaning to a person of ordinary skill in the art and is not to be limited to a special or customized meaning. The term specifically may refer, without limitation, to an elongate object which may be embodied as a surrounding or enclosing structure. The envelope may specifically be a three-dimensional structure encompassing an object such as the medical device at least partially. The distance between a surface of the object and a surface of the envelope may have a constant value. Specifically, the surface of the envelope may be in direct contact with the surface of the object. As used herein, the term "at least partially encompass" may generally refer to a situation in which one object A encloses a circumference of another object B at at least one position or section of the object B.

As outlined above, the envelope is a flexible envelope. The term "flexible envelope" as used herein is a broad term and is to be given its ordinary and customary meaning to a person of ordinary skill in the art and is not to be limited to a special or customized meaning. The term specifically may refer, without limitation, to an arbitrary envelope which may be bendable or deformable by forces which usually occur during holding or stretching of the envelope with both hands and moving the hands relative to each other, such as forces of 10 N or less. Specifically, the flexible envelope may be made of or may contain at least one deformable material, such as at least one plastic or malleable material and/or at least one elastic material. The flexible envelope preferably deforms reversibly and returns at least partially or preferably completely to its original shape when an applied stress is removed.

The term "side" as used herein is a broad term and is to be given its ordinary and customary meaning to a person of ordinary skill in the art and is not to be limited to a special or customized meaning. The term specifically may refer, without limitation, to different surfaces forming an outside of an arbitrary object. The terms "first side" and "second side" may be considered as nomenclature only, without numbering or ranking the named elements, without specifying an order and without excluding a possibility that several kinds of first sides and second sides may be present. Further, additional sides such as one or more third sides may be present. The first side and the second side may specifically respectively refer to longitudinal sides of the flexible envelope. Thus, shorter sides of the flexible envelope may respectively extend transverse, specifically perpendicular, to the first side and the second side. The second side may specifically refer to a front side of the flexible envelope and the first side may specifically refer to a rear side of the flexible envelope. Preferably, in a mounted, i.e. wrapped state of the jacket, specifically of the flexible envelope, wherein the jacket, specifically of the flexible envelope, is fixedly connected to an extremity of the user or the patient, the first side may face a skin site of the user or the patient and the second side may face an outer environment of the user of the patient.

Further, the first side may be at least partially, preferably fully, colored with at least one first color and the second side may be at least partially, preferably fully, colored with at least one second color. The first color may be different from the second color. Thus, a recognition of the first side and the second side by the user or the patient may be facilitated and a handling of the jacket may be improved.

The flexible envelope may comprise at least one substrate. As further used herein, the term "substrate" may refer to an arbitrary element which is suitable to carry one or more other elements disposed thereon or therein. As an example, the substrate may be a flat substrate, such as a substrate having a lateral extension exceeding its thickness by at least a factor of 2, at least a factor of 5, at least a factor of 10, or even at least a factor of 20 or more. The substrate specifically may have an elongated shape. Specifically, the substrate may have rounded ends. More specifically, the substrate may have an oval ground shape. However, other shapes may also be feasible. The first sealing lip and the second sealing lip, which will further be described below in more detail, may be attached to the substrate. Specifically, the first sealing lip and/or the second sealing lip may be fixedly applied to the substrate. As further used herein, the term "fixedly applied" refers to the fact that the first sealing lip and/or the second sealing lip contact the substrate and are mounted onto the substrate in such a way that the first sealing lip and/or the second sealing lip do not come off the substrate without exerting additional forces to the first sealing lip and/or the second sealing lip and/or to the substrate in order to remove these elements from each other. Specifically, the first sealing lip and/or the second sealing lip may be adhered to the substrate by material engagement, such as by directly gluing the first sealing lip and/or the second sealing lip to the substrate. Alternatively, the substrate and one or both of the first sealing lip, the second sealing lip may be manufactured as one single piece.

The flexible envelope, specifically the substrate, may comprise at least one elastic material, specifically at least one material selected from the group consisting of: a rubber including a natural rubber, a latex, a silicone polymer; a silicone copolymer; an elastomer comprising at least one silicone copolymer; an elastomer comprising at least one polyurea copolymer; an elastomer comprising a copolymer of polydimethylsiloxane; an elastomer comprising a copolymer of dimethylsiloxane and urea.

Further, the flexible envelope may comprise at least one low elasticity section. Preferably, the low elasticity section of the flexible envelope covers an area of the medical device, and preferably also an area of the first sealing lip. The low elasticity properties of the low elasticity section of the flexible envelope covering the area of the medical device, and preferably also the area of the first sealing lip, may enable a stable encasing of the medical device at a place where the medical device is positioned on the skin. Preferably, the low elasticity section is less elastic than other sections of the flexible envelope and may possess an elasticity such that this section is only deformed by up to 10%, or up to 5% of the length of the section when stretched parallel to a flexible envelope surface with a force of up to 100 Newton, up to 50 Newton or up to 10 Newton, preferably between 10 to 50 Newton. Other sections of the flexible envelope may possess a high elasticity such that this section is deformed by up at least 10%, at least 25%, at least 50% or at least 75%, at least 100%, or at least 150% of a length of the section when stretched parallel to the flexible envelope surface with a force of up to 100 Newton, up to 50 Newton or up to 10 Newton, preferably between 10 to 50 Newton. Preferably, the low elasticity section may cover an area corresponding to an area being within a radius from a center point of an interior space being enclosed by the first sealing lip. Specifically, the radius may exceed at least a factor of 1.1, preferably of 1.2, more preferably of 1.5, most preferably of 2, of a median distance between the center point of the interior space of the first sealing lip to the first sealing lip.

Moreover, the skin facing first side may at least partially comprise at least one adhesive material. Specifically, an interior area surrounded by the second sealing lip may be covered with the adhesive material. Further, specifically, the first sealing lip and/or the second sealing lip may be covered with the adhesive material. Specifically, sections of the first sealing lip and/or the second sealing lip forming contact areas for the skin site of the user or the patient may be covered with the adhesive material. Moreover, the first side of the envelope contacting sealing end may be covered with the adhesive material to allow the envelope contacting sealing end to fixedly connect to the second side of the flexible envelope. The adhesive material may specifically be a water-insoluble adhesive material. The adhesive material may enable a good adhesion between the jacket and the skin site of the user or the patient, specifically for the process of tensioning the flexible envelope contacting end in order to wrap the flexible envelope contacting end around the extremity of the user or the patient.

The flexible envelope may be at least partially made of at least one transparent material. The transparent material may be at least one optically transparent material. Specifically, the transparent material may be in at least one area covering the medical device during use. Specifically, an area of the flexible envelope may be made of the transparent material. The area may specifically correspond to an area being surrounded by the first sealing lip or all areas of the flexible envelope that cover the area surrounded by the first sealing lip after mounting of the jacket. Thus, the medical device may be visually inspectable while the jacket is mounted on the extremity of the user or the patient. This is beneficial for the mounting/positioning of the Jacket on one hand and for inspecting or reading any visual information displayed on the medical device such as alarms, messages or other information displayed on the medical device, blinking LEDs, etc.

The flexible envelope is configured to be wrapped around the extremity of the user or the patient. The extremity may be selected from the group consisting of: an arm, specifically an upper arm; a stomach; a shoulder; a hip; a back; or a leg. Specifically, the extremity may be the upper arm. The flexible envelope may specifically comprise a length of 200 mm to 2000 mm, preferably of 300 mm to 1500 mm, more preferably of 400 mm to 600 mm. Further, the flexible envelope may comprise a width of 30 mm to 300 mm, preferably of 30 mm to 200 mm, more preferably of 50 mm to 100 mm. Further, the substrate may comprise a thickness of 0.1 mm to 10 mm, preferably of 0.5 mm to 5 mm, more preferably of 1 mm to 3 mm. However, other dimensions may be feasible. The skilled person will appreciate that the dimensions of the flexible envelope generally depend on the size of the extremity and a size and a position of the medical device to be protected with the jacket according to the invention.

The term "sealing lip" as used herein is a broad term and is to be given its ordinary and customary meaning to a person of ordinary skill in the art and is not to be limited to a special or customized meaning. The term specifically may refer, without limitation, to an arbitrary element which is configured to fully or partially concealing at least one opening and/or to fully or partially surround one or more objects to be sealed off from environmental influences such as moisture, dust and/or dirt. Specifically, the sealing lip may be configured to at least partially surround the at least one element to be sealed off from the environmental influences in at least two dimensions.

The terms "first sealing lip" and "second sealing lip" may be considered as nomenclature only, without numbering or ranking the named elements, without specifying an order and without excluding a possibility that several kinds of first sealing lips and second sealing lips may be present. Further, additional sealing lips such as one or more third sealing lips may be present.

The first sealing lip and the second sealing lip may respectively form a protrusion on the flexible envelope, specifically on the substrate. Thus, the first sealing lip and the second sealing lip may respectively protrude from a surface of the flexible envelope, specifically of the substrate.

In a cross-sectional view transverse, specifically perpendicular, to a longitudinal axis of the flexible envelope, specifically to the substrate, the first sealing lip and the second sealing lip may respectively define a cross-sectional profile. The cross-sectional profile may be spherically shaped or may be shaped as an oval. However, other shapes may be feasible. The first sealing lip and/or the second sealing lip may have a height of 1 mm to 100 mm, preferably of 2 mm to 60 mm, more preferably of 5 mm to 30 mm, more preferably of 5 mm to 15 mm. The skilled person will appreciate that the dimensions of the sealing lip generally depend on a size of the flexible envelope, the size of the extremity and the size and the position of the medical device to be protected with the jacket according to the invention. The term "height" may refer to a distance between the surface of the substrate and a point of the first sealing lip or of the second sealing lip being furthest from the surface of the substrate in the cross-sectional profile. Specifically, a first sealing lip height of the first sealing lip may be identical to a second sealing lip height of the second sealing lip. However, alternatively, the first sealing lip height of the first sealing lip may differ from the second sealing lip height of the second sealing lip. Specifically, the first sealing lip height and/or the second sealing lip height may respectively be constant, with a tolerance of variation of e.g. no more than 20% or no more than 10%. However, other embodiments are feasible. Further, the first sealing lip and/or the second sealing lip may respectively be circumferential sealing lips. Thus, the first sealing lip and/or the second sealing lip may respectively define a closed sealing line on the flexible envelope, specifically on the substrate.

One or both of the first sealing lip, the second sealing lip may comprise at least one elastic material. Specifically, one or both of the first sealing lip, the second sealing lip may be manufactured from at least one material selected from the group consisting of: a rubber including a natural rubber; a latex; a silicone polymer; a silicone copolymer; an elastomer comprising at least one silicone copolymer; an elastomer comprising at least one polyurea copolymer; an elastomer comprising a copolymer of polydimethylsiloxane; an elastomer comprising a copolymer of dimethylsiloxane and urea.

As outlined above, the first sealing lip and the second sealing lip are respectively configured to surround the medical device. The second sealing lip additionally surrounds the first sealing lip. The term "surrounding an object" as used herein is a broad term and is to be given its ordinary and customary meaning to a person of ordinary skill in the art and is not to be limited to a special or customized meaning. The term specifically may refer, without limitation, to a property of a first object of being embodied and arranged relative to a second object such that the second object is enclosed at least partially, preferably fully, in at least two dimensions by the first object. Thus, the first object may encircle the second object in the at least two dimensions. Specifically, the first sealing lip and the second sealing lip, respectively, may be configured to surround the medical device laterally. Thus, the first sealing lip and the second sealing lip, respectively, may be situated at a side of the medical device. Thus, the first sealing lip and the second sealing lip, respectively, may encase or encircle the medical device. The first sealing lip and the second sealing lip, respectively, may be circumferential sealing lips as described above.

In a top view, the first sealing lip may have a shape selected from the group consisting of: a circular shape, an oval shape, a polygon shape, a rectangular shape. However, other shapes may be feasible as well. Preferably, the shape of the first sealing lip is based on an outer shape of the medical device when the latter is attached to the skin and viewed from the top. Preferably, the first sealing lip is in contact with the outer shape of the medical device during use. Alternatively, there may be a lateral distance between an outer margin of the medical device and an inner margin of the first sealing lip of up to 10 mm, preferably of up to 5 mm. The first sealing lip, in the top view, may have a length of less than 120 mm, or less than 100 mm, such as a length of less than 50 mm, less than 30 mm, less than 20 mm or, e.g. a length of 10 mm to 40 mm. Further, the first sealing lip, in the top view, may have a width of less than 120 mm, less than 100 mm, less than 80 mm, less than 60 mm, less than 40 mm, less than 30 mm, such as a width of 10 to 30 mm.

As used herein, the term "height" may refer to a distance between two longitudinal surfaces of the medical device. Specifically, the medical device may have a medical device height. In the mounted state of the jacket, wherein the first sealing lip may be in a compressed state, the first height of the sealing lip is preferably larger than the medical device height. This way the sealing of the medical device from the environment is improved. Thus, in the mounted state of the jacket, wherein the flexible envelope is wrapped around the extremity of the user or the patient, the medical device may be enclosed on one longitudinal side by the skin site of the user or the patient, on one other longitudinal side of the medical device by at least one surface of the flexible envelope, specifically of the substrate, and laterally on the shorter sides by the first sealing lip. This way a tight sealing of the medical device may be accomplished while at the same time ensuring that the medical device can continue to operate in a location on the skin where it has been placed. This arrangement is particularly advantageous for all medical devices worn or attached to the skin and in particular for transcutaneous devices because the medical device, specifically including the transcutaneously positioned part of the device, can be left in place when applying the jacket and also when removing the jacket. The versatile and easy to mount jacket of the invention thus allows for ergonomic and swift mounting and removal of the jacket and, moreover, avoids the necessity to dismount and remount the medical device for the purpose of applying the jacket as it is the case with the medical device covers available in the prior art. Therefore, wearing comfort, safety of use, specifically protection of the medical device against humidity and water contact, is provided by the jacket of the present invention. At the same time the tight wrapping of the jacket also ensures that the medical device remains in place to safeguard proper operation. Finally, as the user can adapt the tightness of the jacket's wrapping around the extremity, the user may optimize the mounting of the jacked according to his wearing preferences while avoiding uncomfortable wearing conditions known from prior art devices.

The first sealing lip together with the first side of the substrate may form an open compartment for the medical device. The term "compartment" as used herein is a broad term and is to be given its ordinary and customary meaning to a person of ordinary skill in the art and is not to be limited to a special or customized meaning. The term specifically may refer, without limitation, to an arbitrary interior space of an object. Specifically, the interior space may receive, hold or harbor the medical device. The interior space may be configured for receiving, holding or harboring another apparatus, such as, e.g. an infusion set that is in fluid connection with the medical device being a pump, the infusion set being attached to the skin and comprising a needle which may infuse a drug into the body of the patient. The term "open compartment" as used herein is a broad term and is to be given its ordinary and customary meaning to a person of ordinary skill in the art and is not to be limited to a special or customized meaning. The term specifically may refer, without limitation, to an arbitrary compartment which is accessible from at least one side of the compartment. Exemplarily, the compartment may have several sidewalls and a bottom. However, a top side of the compartment may be open. Thus, the top side may be referred to as open side.

As outlined above, the second sealing lip is spaced apart from the first sealing lip. The term "being spaced apart from something" as used herein is a broad term and is to be given its ordinary and customary meaning to a person of ordinary skill in the art and is not to be limited to a special or customized meaning. The term specifically may refer, without limitation, to an arrangement of one element relative to another element, wherein the two elements are arranged in a distance to each other. Thus, the one element and the another element may be arranged in a contactless manner. Specifically, the first sealing lip and the second sealing lip may be arranged in a distance to each other in one level, particularly in one level of the substrate. Specifically, the second sealing lip preferably surrounds the first sealing lip. Thus, the second sealing lip may enclose, specifically encircle, the first sealing lip. In the top view of the flexible envelope, the first sealing lip may have a first median diameter and the second sealing lip may have a second median diameter. The first median diameter may be smaller than the second median diameter.

Specifically, the second sealing lip may comprise at least one first portion and at least one second portion. The terms "first portion" and "second portion" may be considered as nomenclature only, without numbering or ranking the named elements, without specifying an order and without excluding a possibility that several kinds of first portions and second portions may be present. Further, additional portions such as one or more third portions may be present.

The term "portion" as used herein is a broad term and is to be given its ordinary and customary meaning to a person of ordinary skill in the art and is not to be limited to a special or customized meaning. The term specifically may refer, without limitation, to an arbitrary component of an object. The component may be configured for interacting with a further component of the object. Specifically, the first portion and the second portion may be capable of interacting with each other, such as in order to perform one or more purposes, such as in order to shield an interior space enclosed by the first portion and by the second portion.

The second portion may be configured to establish a form-fit connection with at least one section of the first portion, specifically when the jacket is in the mounted state. The term "form-fit connection" may generally refer to a connection between two or more components via mechanical interlocking. Specifically, the first portion may have a first portion shape and the second portion may have a second portion shape and the first portion shape and the second portion shape may be complementary to each other. Specifically, the first portion may be a skin contacting portion. Specifically, a skin facing side of the first portion may be the skin contacting portion. The first portion may form a skin contacting sealing lip. The first portion may be configured for contacting the skin site of the user or the patient. The second portion may be an interlocking portion. Specifically, a skin facing side of the second portion may be the interlocking portion. The second portion may form an interlocking sealing lip. The second portion may be configured for contacting the second side of the flexible envelope and for being arranged next to the first portion of the second sealing lip thereby establishing a form-fit connection. Thus, the second portion may be configured to enclose the first portion at least partially. The form-fit connection may provide a sealing, specifically a watertight sealing of an interior enclosed by the form-fit connector of the first portion and the second portion. To the contrary, an attachment of the contacting sealing end to the second side may ensure that the jacked, specifically in the wrapped state, may remain in place.

Specifically, the first portion and the second portion may respectively comprise at least one magnetic material. The first portion and the second portion may respectively have a supporting surface being configured for contacting a surface of another object such as the skin site of the user or the patient in case of the first portion and such as the second side of the flexible envelope in case of the second portion. Further, the first portion and the second portion may respectively have lateral surfaces neighboring the supporting surface. More specifically a first lateral surface of the first portion may comprise at least one first magnetic material and a second lateral surface of the second portion of the flexible envelope may comprise at least one complementary second magnetic material, preferably of opposite magnetic polarity than the first magnetic material. Thus, the first magnetic material and the second magnetic material may improve a form closure, specifically during mounting the jacket on the extremity of the user or the patient. Further, the first magnetic material and the second magnetic material may improve a correct placing of the jacket onto the extremity of the user or the patient, specifically relative to the medical device.

The skin contacting side of first end of the jacket may be coated with an adhesive which adheres to the skin. This makes it easier for the user or patient to mount the jacket one handed or without help of another person.

Specifically, the first portion may at least partially run along an outer edge of the flexible envelope. The first portion may be aligned with the outer edge of the flexible envelope. However, alternatively, the first portion may run along a distance to the outer edge of the flexible envelope. The first portion may cover 50% to 80 % of the outer edge, specifically 60% to 70 % of the outer edge. The first portion may have at least two first portion ends. Specifically, the first portion ends of the first portion may be located on opposing sides relative to a longitudinal axis of the flexible envelope. An end of the flexible envelope opposing the envelope contacting sealing end may have the first portion. The flexible envelope contacting sealing end may be free from the first portion.

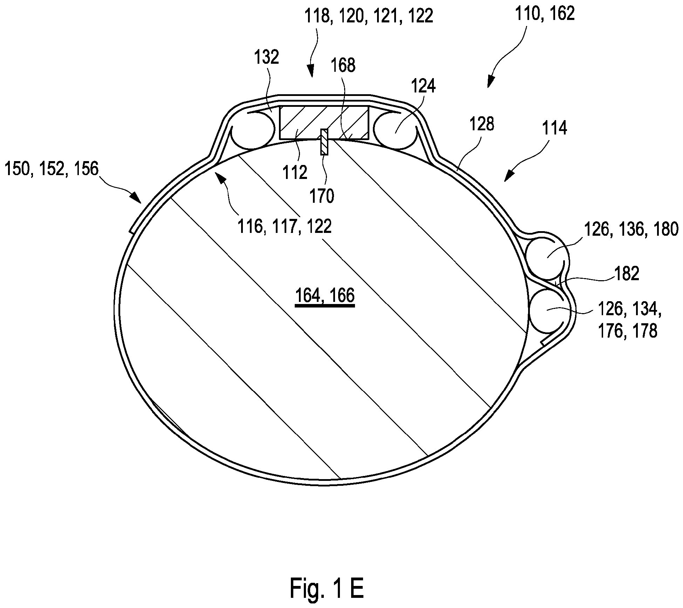

The second portion may extend transverse to a direction of extension of the first portion. Further, the second portion may extend transverse to a direction of extension of the flexible envelope, specifically of the substrate. Specifically, in the top view of the flexible envelope, the second portion may have a curved shape, specifically an arch-shape. However, other shapes may be feasible. As the skilled person will appreciate from the teaching throughout the specification and the figures the shape of the flexible envelope and the respective portions of the second sealing lip will preferably be of such a shape that after mounting the jacket the interlocking portion of the second sealing lip will interlock with the first portion of the second sealing lip to improve the sealing of the jacket as evident, e.g. from Figure 1E.

The second portion may have at least two second portion ends. Specifically, the first portion ends of the first portion may respectively contact the second portion ends of the second portion. Specifically, the first portion ends of the first portion may respectively be firmly bonded to the second portion ends of the second portion. Thus, the first portion and the second portion of the second sealing lip may be separated portions or parts. However, other embodiments may be feasible. Thus, the first portion and the second portion may be manufactured as one single piece. Specifically, the first portion and the second portion may form a continuous, specifically fused, part of the first portion ends and the second portion ends.

Further, one or both of the first sealing lip and the second sealing lip may be inflatable sealing lips. Thus, the first sealing lip and/or the second sealing lip may be manufactured as hollow components of the flexible envelope. The first sealing lip and/or the second sealing lip may specifically be configured to be inflatable with air. Specifically, both of the first sealing lip and the second sealing lip may be inflatable sealing lips and the first sealing lip and the second sealing lip may be fluidically connected to each other via a channel of the flexible envelope. The channel may form a connection between the first sealing lip and the second sealing lip. Specifically, the channel may have two ends, wherein one of the two ends leads to a point of the first sealing lip and wherein the other one of the two ends leads to a point of the second sealing lip. One or both of the first sealing lip and the second sealing lip may be fillable via a lockable mouthpiece of the jacket. The lockable mouthpiece may specifically be located on one of the shorter sides of the flexible envelope or on the second side of the flexible envelope. Thus, the jacket with inflatable sealing lips may be associated with the advantage that the jacket may be transportable and storable by the user or the patient in a space-saving manner when stored in a non-inflated condition. Alternatively, the jacket with the inflatable sealing lips may be fabricated and sold in inflated condition. Alternatively, one or both of the first sealing lip and the second sealing lip may be filled with a compressible gel or material such as agar-agar, silicon or the like. Thus, one or both of the first sealing lip and the second sealing lip may be compressible sealing lips. The term "compressible" may generally refer to a property of an object or of a material to change a relative volume as a response to a pressure change, specifically a stress change. Specifically, the change of the relative volume may refer to a volume reduction. Specifically, a density of the material may reduce.

The term "end" as used herein is a broad term and is to be given its ordinary and customary meaning to a person of ordinary skill in the art and is not to be limited to a special or customized meaning. The term specifically may refer, without limitation, to a last part of an arbitrary elongate element neighboring an outer environment of the element. In a top view of the element, the end may correspond to a maximum of 50% of a length of the element starting from an outer edge of the element up to a middle portion of the element. Specifically, in the top view of the element, the end may correspond to a maximum of 30%, preferably of 20%, more preferably of 10%, of a length of the element starting from the outer edge of the element up to an interior portion of the element. The term "envelope contacting sealing end" as used herein is a broad term and is to be given its ordinary and customary meaning to a person of ordinary skill in the art and is not to be limited to a special or customized meaning. The term specifically may refer, without limitation, to an end of the flexible envelope which is configured to get in contact with a part of the flexible envelope which is different from the envelope contacting sealing end. Specifically, the envelope contacting sealing end may be fixedly connectable to the part of the flexible envelope as will further be described below in more detail. Specifically, in the top view of the flexible envelope, the flexible envelope may have two opposing ends. One end may have at least parts of the second sealing lip, specifically at least parts of the first portion of the second sealing lip. The other end may correspond to the envelope contacting sealing end.

As outlined above, the envelope contacting sealing end is fixedly connectable to the second side of the flexible envelope. The term "connectable" as used herein is a broad term and is to be given its ordinary and customary meaning to a person of ordinary skill in the art and is not to be limited to a special or customized meaning. The term specifically may refer, without limitation, to a property of an object to be placeable into and fixable in close physical proximity to another object. In particular, the object that is connectable to another object may be in direct or indirect physical contact with the other object when it is connected to it. The term "fixedly connectable" as used herein is a broad term and is to be given its ordinary and customary meaning to a person of ordinary skill in the art and is not to be limited to a special or customized meaning. The term specifically may refer, without limitation, to a state of at least one object and at least one other object wherein the object and the other object are in contact with each other in such a way that the object does not come off the other object or vice versa without exerting additional forces to the object or the other object in order to remove these objects from each other.

Specifically, the envelope contacting sealing end is couplable with the second side of the flexible envelope. The term "couplable" as used herein is a broad term and is to be given its ordinary and customary meaning to a person of ordinary skill in the art and is not to be limited to a special or customized meaning. The term specifically may refer, without limitation, to a property of one or more components which are configured to be brought from a disconnected state, also referred to as a non-mated state, into a connected state, also referred to as a mated state or referred to as a coupled state, and back into the disconnected state. In the disconnected state, the components may be completely disassembled, e.g. the components may be arranged in a distance to each other and may not be in contact with each other. However, alternatively, in the disconnected state, a contact between the components may remain.

Further, the envelope contacting sealing end may be releasably or ir-releasably connectable with the second side of the flexible envelope. As further used herein, the term "releasable", in the context of a mechanical connection, refers to the fact that the mechanical connection may be brought from a disconnected state, also referred to as a non-mated state, into a connected state, also referred to as a mated state, and back into the disconnected state at least once, preferably several times. Thus, the mechanical connection may be closed and released at will. Specifically, the mechanical connection may be releasable without using any tools, simply by manual action. As an example, for opening a connection between the envelope contacting sealing end and the second side of the flexible envelope forces of no more than 50 N, such as of no more than 20 N, such as of no more than 10 N, may be required. The force may be applied by one hand or even the fingers or fingertips of the user or the patient. Specifically, the envelope contacting sealing end may be fixedly connectable to the second side by an adhesive material. The adhesive material may be located at the first side of the flexible envelope, specifically in a region of the envelope contacting sealing end and the adhesive material may be configured to be attached to the second side of the flexible envelope. Specifically, the adhesive material may be configured to be attachable to and detachable from a surface several times.

Specifically, the envelope contacting sealing end may be made of a material which is more elastic, preferably stretchable, than a material of a remaining portion of the flexible envelope. The term "elastic" may generally refer to a property of an arbitrary object being made of a material of being deformable by up at least 10%, at least 25%, at least 50% or at least 75%, at least 100%, or at least 150% of a dimension, such as a length of at least one section of the object when stretched parallel to a surface with a force of up to 100 Newton, up to 50 Newton or up to 10 Newton, preferably between 10 to 50 Newton. Thereby, the object may return to an original size and shape when the force is removed. The term "stretchable" may generally refer to a property of an arbitrary element of being elongatable in at least one direction. Specifically, the envelope contacting sealing end may be stretchable in a direction of extension of the flexible envelope by at least 10%, preferably by at least 25%, more preferably by at least 50%, more preferably by at least 75%, more preferably by at least 100% and most preferably by at least 150% of an initial length of the envelope contacting sealing end. Further, the remaining portion may be stretchable in the direction of extension of the flexible envelope by at least 10%, preferably by at least 25%, more preferably by at least 50%, more preferably by at least 75%, more preferably by at least 100% and most preferably by at least 150% of an initial length of the envelope contacting sealing end.

Further, additionally or alternatively, the envelope contacting sealing end may be fixedly connectable to the second side by a hook and loop fastener. The term "hook and loop fastener" may generally refer to an assembly of at least a first component and at least a second component wherein a first surface of the first component is attachable to an opposing second surface of the second component. The first surface may comprise a plurality of small hooks, and the second surface may comprise a plurality of small loops or vice versa. In case the first surface and the second surface are pressed together, the hooks may catch in the loops. As a consequence, the first component and the second component may be fixedly connectable to each other. However, the first component may be disconnectable from the second component such as by pulling or peeling the first surface and the second surface apart from each other. Thus, the hook and loop fastener may also be referred to as a hook and pile fastener or as a touch fastener. Specifically, the envelope contacting sealing end may have an area on the first side of the flexible envelope comprising hooks and an area on the second side of the flexible envelope may comprise loops, or vice versa. Preferably, the reversible or irreversible fixed connection is formed by a means for fixedly connecting known to the skilled person such as Velcro, an adhesive, magnets, zip fastening, hooks, eyes, etc.

Further, additionally or alternatively, the envelope contacting sealing end may comprise at least one third sealing lip. Specifically, the third sealing lip may be located on the skin facing first side of the flexible envelope. More specifically, the third sealing lip may run along an outer edge of the flexible envelope or may run parallel to the second sealing lip but may be positioned between the first sealing lip and second sealing lip. The third sealing lip may be located at the envelope contacting sealing end. Further, ends of the third sealing lip may be in direct contact with the first portion ends of the first portion of the second sealing lip. Specifically, the third sealing lip and the second sealing lip may be formed as a single piece. The third sealing lip may be configured to contact the second side of the flexible envelope. The introduction of the third sealing lip may introduce a further sealing barrier to prevent agents such as water or humidity to reach the medical device.

Moreover, the jacket may additionally comprise at least one humidity sensor. The term "humidity sensor" as further used herein may specifically refer to a sensor which is configured to detect a presence of humidity in an environment. Specifically, the humidity sensor may be configured to detect a water intrusion in the environment. In case the presence of humidity is detected, a warning signal may be issued. Specifically, the humidity sensor may be arranged between the first sealing lip and the second sealing lip. Thus, the humidity sensor may be arranged within an area of the flexible envelope being surrounded by the second sealing lip. Further, the humidity sensor may be arranged within the first sealing lip. Thus, the humidity sensor may be arranged within an area of the flexible envelope being surrounded by the first sealing lip. Specifically, the humidity sensor may comprise at least two electrodes. In case an electrically conductive connection between the two electrodes is formed such as via an electrically conductive fluid such as water, an electrical signal may be generated which can then trigger an acoustic, vibrational or visual alarm. However, other embodiments of the humidity sensor may also be generated. Further, the humidity sensor may have at least one signal transducer.

The jacket may further comprise a means to absorb humidity such as silica, which can absorb water or humidity in the event that water or humidity enters the jacket during use. Such means to absorb humidity may be positioned on the substrate on the first side between the first and the second sealing lip, preferably in the shape of a closed ring.

In a further aspect of the present invention, a method for watertight sealing a medical device is disclosed.

The method comprises the method steps as given in the independent claims and as listed as follows. The method steps may be performed in the given order. However, other orders of the method steps are feasible but not encompassed by the claims. Further, one or more of the method steps may be performed in parallel and/or on a timely overlapping fashion. Further, one or more of the method steps may be performed repeatedly. Further, additional method steps may be present which are not listed.

The method comprises the following steps:
a) mounting at least one medical device on a skin site of an extremity of a user;
b) placing the jacket as described above or as will further be described below in more detail onto the skin site such that the first sealing lip and the second sealing lip surround the medical device; and
c) wrapping the flexible envelope around the extremity and fixedly connecting the envelope contacting sealing end to the opposing second side of the of flexible envelope.

The extremity may be selected from the group consisting of an arm, specifically an upper arm; a stomach; a shoulder; a back; a hip; a leg. However, other extremities may be feasible. The wrapping of the flexible envelope around the extremity may specifically occur via tensioning the envelope contacting sealing end. The method step b) may further comprise a smoothing of the first sealing lip on the skin site of the user or the patient. Thus, the first sealing lip may form a close connection to the skin site. The method step c) may comprise a forming of the form-fit connection such as described above or as will further be described below in more detail.

The proposed jacket for watertight sealing a medical device and the proposed method for watertight sealing a medical device provide many advantages over known devices and methods. Thus, the jacket for watertight sealing a medical device according to the present invention may specifically be cheap and may be easily mounted. Specifically, the patient does not need any help to mount the jacket. Additionally, the jacket may be configured to serve to better fix the medical device on the skin site of the patient.

The flexible envelope may be configured to be wrapped around the extremity of the patient. The first sealing lip and the second sealing lip may respectively have skin contact and, thus, may avoid an ingress of water or other fluids. The skin contacting side of first end of the jacket may be coated with an adhesive which adheres to the skin. This makes it easier for the user or patient to mount the jacket one handed or without help of another person. The envelope contacting sealing end may be used for fixing the flexible envelope to the skin site of the patient. The envelope contacting sealing end may adhere to the envelope.

The proposed jacket for watertight sealing a medical device may be suitable for encasing the medical device which may be worn on the extremities of the user or the patient. The jacket may be configured for providing a protection for the medical device, specifically against humidity and water. Preferably, the jacket provides a sealing which prevents water from contacting the medical device during use of the jacket if the water source is splash water, rain, a shower, a bath, or swimming. In case of swimming the sealing protects from water to contact the medical device at a swimming depth of up to 3 meter water depth, or up to 1 meter water depth; preferably for a swimming time of up to 5 min, up to 10 min, up to 20 min or up to 30 minutes.

As outlined above, in the mounted state of the jacket, wherein the first sealing lip may be in a compressed state, the first height of the sealing lip is preferably larger than the medical device height. Thus, the sealing of the medical device from the environment may be improved. Specifically, a tight sealing of the medical device may be accomplished while at the same time ensuring that the medical device can continue to operate in a location on the skin where it has been placed. This arrangement is particularly advantageous for all medical devices worn or attached to the skin and in particular for transcutaneous devices because the medical device, specifically including the transcutaneously positioned part of the device, can be left in place when applying the jacket and also when removing the jacket. The versatile and easy to mount jacket of the invention thus allows for ergonomic and swift mounting and removal of the jacket and, moreover, avoids the necessity to dismount and remount the medical device for the purpose of applying the jacket as it is the case with the medical device covers available in the prior art. Therefore, wearing comfort, safety of use, specifically protection of the medical device against humidity and water contact, is provided by the jacket of the present invention. At the same time the tight wrapping of the jacket also ensures that the medical device remains in place to safeguard proper operation. Finally, as the user can adapt the tightness of the jacket's wrapping around the extremity, the user may optimize the mounting of the jacked according to his wearing preferences while avoiding uncomfortable wearing conditions known from prior art devices.

### Short description of the Figures

Further optional features and embodiments will be disclosed in more detail in the subsequent description of embodiments, preferably in conjunction with the dependent claims. Therein, the respective optional features may be realized in an isolated fashion as well as in any arbitrary feasible combination, as the skilled person will realize. The scope of the invention is not restricted by the preferred embodiments. The embodiments are schematically depicted in the Figures. Therein, identical reference numbers in these Figures refer to identical or functionally comparable elements.

In the Figures:
- Figures 1A to 1E: show an exemplary embodiment of a jacket according to the present invention in a top view (Figure 1A) and in various cross-sectional views (Figures 1B to 1E); and
- Figures 2A to 2B: show a further exemplary embodiment of a jacket according to the present invention in a top view (Figure 2A) and in a cross-sectional view (Figure 2B).

### Detailed description of the embodiments

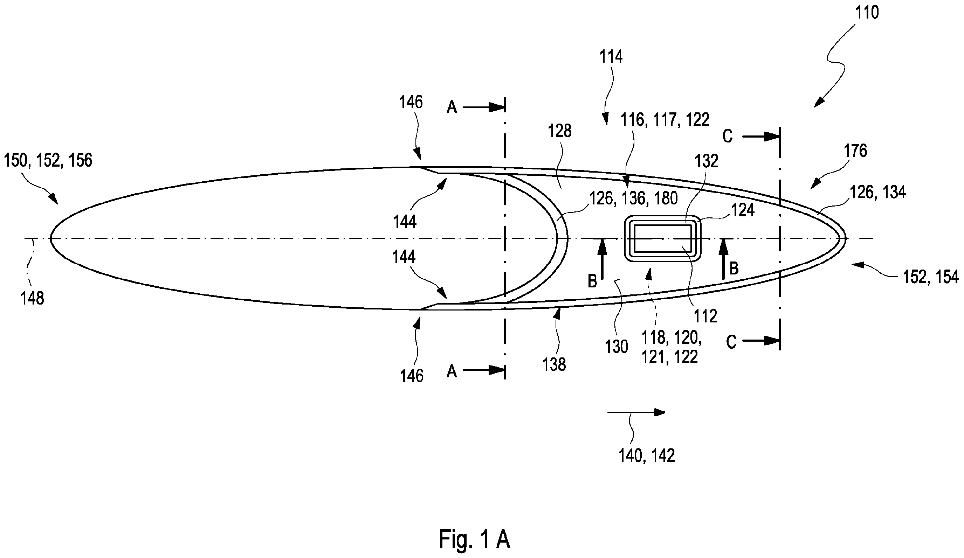

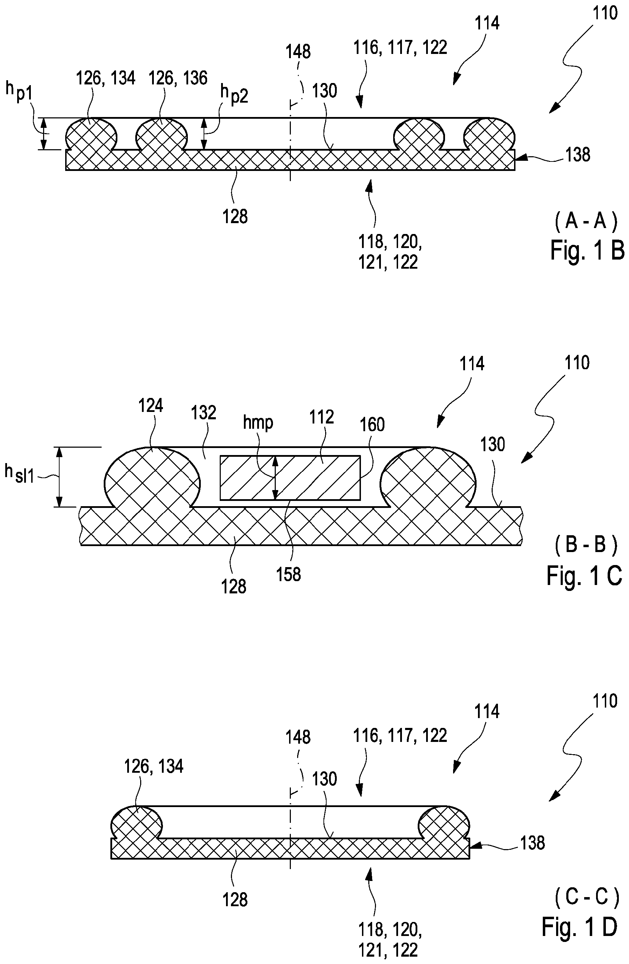

Figures 1A to 1E show an exemplary embodiment of a jacket 110 according to the present invention in a top view (Figure 1A) and in various cross-sectional views (Figures 1B to 1E).

Figure 1A shows a jacket 110 for watertight sealing a medical device 112 in a top view in a state before mounting. The medical device 112 is also depicted in Figure 1A. However, the medical device 112 itself may not be part of the jacket 110. The jacket 110 comprises at least one flexible envelope 114. The flexible envelope 114 comprises at least one first side 116, specifically at least one skin facing first side 117, and at least one second side 120, specifically at least one opposing second side 118 which may specifically face away from the skin. Thus, the second side 120 may also be referred to as outside facing second side 121. In the top view as illustrated in Figure 1A, the skin facing first side 117 is shown. The skin facing first side 117 and the opposing second side 118 may respectively correspond to longitudinal sides 122 of the flexible envelope 114. The flexible envelope 114 may specifically have an elongate shape such as an oval shape. However, other shapes may also be feasible. Further, the flexible envelope 114 may be made at least partially of at least one elastic material.

The flexible envelope 114 comprises at least one first sealing lip 124 configured to surround the medical device 112 and at least one second sealing lip 126 configured to surround the first sealing lip 124 and the medical device 112. The second sealing lip 126 is spaced apart from the first sealing lip 124. Specifically, the flexible envelope 114 may comprise at least one substrate 128 and the first sealing lip 124 and the second sealing lip 126 may respectively be attached to the substrate 128 such as by being adhered to a surface 130 of the substrate 128.

In the top view as illustrated in Figure 1A, the first sealing lip 124 may have a rectangular shape. However, other shapes may be feasible such as a shape close or identical to a top view shape of the medical device. The first sealing lip 124 may form an open compartment 132 for the medical device 112.

Specifically, the second sealing lip 126 may comprise a first portion 134 and a second portion 136. The first portion 134 may at least partially run along an outer edge 138 of the flexible envelope 114. Specifically, the first portion 134 may be aligned with the outer edge 138 of the flexible envelope 114. The second portion 136 may extend transverse to a first direction of extension 140 of the first portion 134. Further, the second portion 136 may extend transverse to a second direction of extension 142 of the flexible envelope 114. The first direction of extension 140 of the first portion 134 may correspond to the second direction of extension 142 of the flexible envelope 114. Specifically, in the top view of the flexible envelope 114 such as illustrated in Figure 1A, the second portion 136 may have an arch-shape. The second portion 136 may have at least two second portion ends 144. The first portion 134 may have at least two first portion ends 146. The first portions ends 146 of the first portion 134 may respectively be firmly bonded to the second portion ends 144 of the second portion 136. The first portion ends 146 of the first portion 134 may be located on opposing sides relative to a longitudinal axis 148 of the flexible envelope 114.

The flexible envelope 114 further comprises at least one envelope contacting sealing end 150. The skin facing first side 117 of the envelope contacting sealing end 150 is fixedly connectable to the outside facing second side 121 of the flexible envelope 114 such as during mounting of the jacket 110. Specifically, in the top view of the flexible envelope 114 as illustrated in Figure 1A, the flexible envelope 114 may have two opposing ends 152. One first end 154 may have at least parts of the second sealing lip 126, specifically at least parts of the first portion 134 of the second sealing lip 126. One second end 156 may correspond to the envelope contacting sealing end 150.

In Figure 1B a cross-sectional view through line A-A as illustrated in Figure 1A is shown. The cross-sectional view shows the second sealing lip 126, specifically the first portion 134 and the second portion 136 of the second sealing lip. In the cross-sectional view as illustrated in Figure 1B, the second sealing lip 126 may have a round ground shape. Specifically, a first portion height hₚ₁ of the first portion 134 may be identical to a second portion height hₚ₂ of the second portion 136.

In Figure 1C a cross-sectional view through line B-B as illustrated in Figure 1A is shown. The cross-sectional view shows the first sealing lip 124. As outlined above, the first sealing lip 124 surrounds the medical device 112. Specifically, the first sealing lip 124 may form the open compartment 132 for the medical device 112. Thus, the medical device 112 may be enclosed on one longitudinal side 158 of the medical device 112 by the surface 130 of the flexible envelope 114. On shorter sides 160 of the medical device 112 the medical device 112 may be enclosed by the first sealing lip 124. The medical device 112 may have a medical device height hₘₚ. In a compressed state after mounting the jacket the medical device height hₘₚ may be smaller than a first sealing lip height hₛₗ₁ of the first sealing lip 124.

In Figure 1D a cross-sectional view through line C-C as illustrated in Figure 1A is shown. The cross-sectional view shows the second sealing lip 136, specifically the first portion 134 of the second sealing lip 136. As can be seen in Figure 1D, the second sealing lip 136 may be aligned with the outer edge 138 of the flexible envelope 114.

In Figure 1E a further cross-sectional view of the jacket 110 is shown. In Figure 1E, the jacket 110 is shown in a mounted state 162. Thus, Figure 1E shows a cross-sectional view of an extremity 164, specifically of an arm 166, of a user or a patient wearing a medical device 112 attachable to the skin, wherein the jacket 110 is wrapped around the extremity 164.

The medical device 112 may be attached to a skin site 168 of the extremity 164. Specifically, the medical device 112 may have an insertable element 170. The insertable element 170 may at least partially be located within or under the skin of the extremity 164.

The flexible envelope 114 may encircle the extremity 164 fully. Thereby, the first sealing lip 124 may surround the medical device 112. The second portion 136 may be configured to establish a form-fit connection with at least one section 176 of the first portion 134. Specifically, the first portion 134 may be a skin contacting portion 178 configured for contacting the skin site 168. The second portion 136 may be an interlocking portion 180 configured for contacting the second side 120 of the flexible envelope 114 and for being arranged next to the first portion 134, specifically next to a lateral surface 182 of the first portion, thereby establishing the form-fit connection. The skin facing first side 117 of the envelope contacting sealing end 150 is fixedly connectable to the outside facing second side 120 of the flexible envelope.

Thus, as illustrated in Figure 1E, when wrapping the flexible envelope 114 around the extremity 164, the second portion 136 of the second sealing lip may be pulled over the first sealing lip 124 which contacts the skin site 168. Thereafter, the envelope contacting sealing end 150 may further be wrapped around the extremity 164 such that the envelope contacting sealing end 150 covers the medical device 112 and the first sealing lip 124. Specifically, the envelope contacting sealing end 150 may be wrapped around the extremity on a part of the flexible envelope 114 opposite the medical device 112. An interlocking of the second portion 136 with the first portion 134 may take place. Thereafter the envelope contacting sealing end 150 may be fixedly attached to the second side of the flexible envelope 114.Thus, the envelope contacting sealing end 150 may be laid up on the second side 120 of the flexible envelope 114.

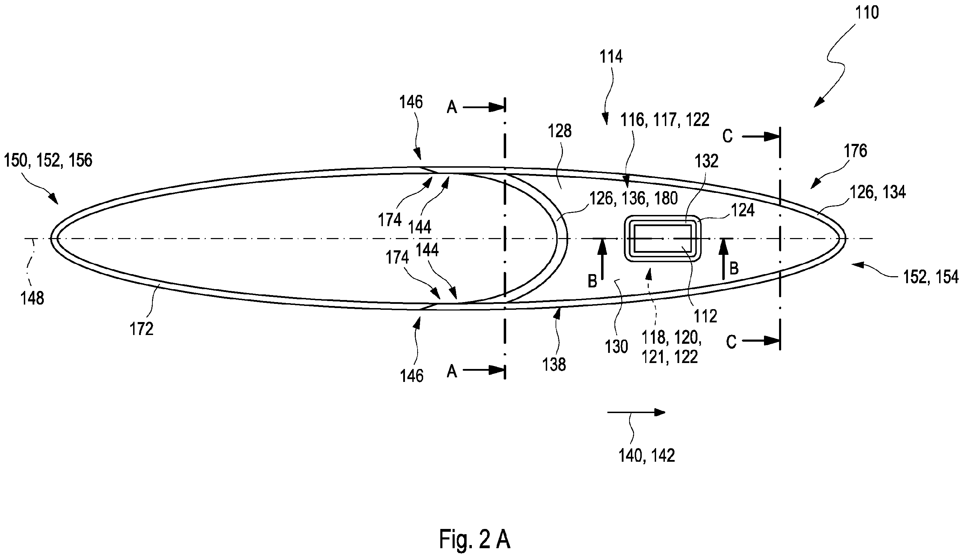

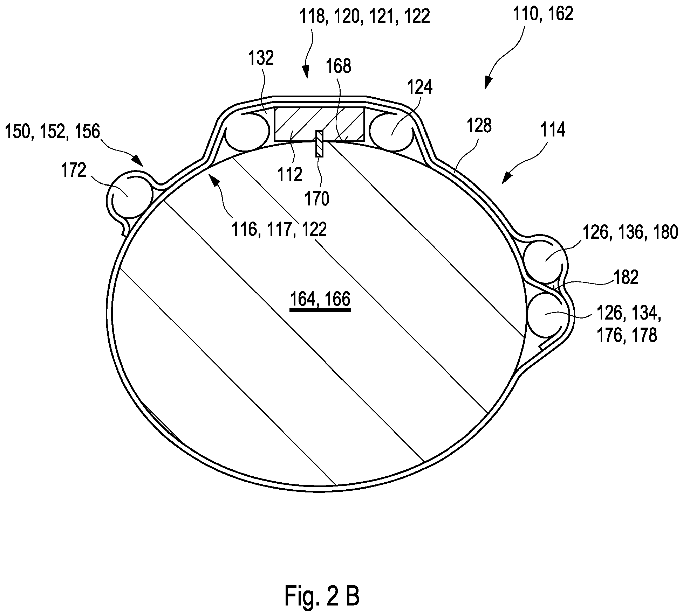

Figures 2A to 2B show a further exemplary embodiment of a jacket 110 according to the present invention in a top view (Figure 2A) and in a cross-sectional view (Figures 2B). The jacket 110 as illustrated in Figures 2A and 2B correspond at least partially to the jacket 110 as illustrated in Figures 1A to 1E. Thus, reference can be made to the description of Figures 1A to 1E above.

As illustrated in Figures 2A and 2B, the first side 116, specifically the envelope contacting sealing end 150, may comprise at least one third sealing lip 172. The third sealing lip 172 may run along the outer edge 138 of the flexible envelope 114. Further, third ends 174 of the third sealing lip 172 may be in direct contact with the first ends 154 of the first portion 134 of the second sealing lip 126. Specifically, the third sealing lip 172 and the second sealing lip 126 may be formed as a single piece. As illustrated in Figure 2B, the third sealing lip 172 may be configured to contact the second side 120 of the flexible envelope 114.

### List of reference numbers

- 110: jacket
- 112: medical device
- 114: flexible envelope
- 116: first side
- 117: skin facing first side
- 118: opposing second side
- 120: second side
- 121: outside facing second side
- 122: longitudinal side
- 124: first sealing lip
- 126: second sealing lip
- 128: substrate
- 130: surface
- 132: open compartment
- 134: first portion
- 136: second portion
- 138: outer edge
- 140: first direction of extension
- 142: second direction of extension
- 144: second portion end
- 146: first portion end
- 148: longitudinal axis
- 150: envelope contacting sealing end
- 152: end
- 154: first end
- 156: second end
- 158: longitudinal side
- 160: shorter side
- 162: mounted state
- 164: extremity
- 166: arm
- 168: skin site
- 170: insertable element
- 172: third sealing lip
- 174: third end
- 176: section
- 178: skin contacting portion
- 180: interlocking portion
- 182: lateral surface

## Claims

1. A jacket (110) for watertight sealing a medical device (112), wherein the jacket (110) comprises at least one flexible envelope (114), wherein the flexible envelope (114) comprises at least one first side (116, 117) for facing the skin of a patient and at least one opposing second side (120), wherein the first side (116) comprises:
• at least one first sealing lip (124), wherein the first sealing lip (124) is configured to surround the medical device (112); and
• at least one second sealing lip (126), wherein the second sealing lip (126) is configured to surround the medical device (112), wherein the second sealing lip (126) is spaced apart from the first sealing lip (124);
wherein the flexible envelope (114) further comprises at least one envelope contacting sealing end (150), wherein the skin-facing first side (117) of the envelope contacting sealing end (150) is fixedly connectable to the opposing second side (120) of the flexible envelope (114) when wrapping the flexible envelope around an extremity of a user,
wherein the second sealing lip (126) surrounds the first sealing lip (124).

2. The jacket (110) according to claim 1, wherein the flexible envelope (114) comprises at least one low elasticity section.

3. The jacket (110) according to any one of claims 1 or 2 wherein the medical device (112) has a medical device height h_{md}, wherein, in a compressed state of the first sealing lip (124), the first sealing lip (124) has a first sealing lip height, wherein the first sealing lip height is larger than the medical device height h_{md}.

4. The jacket (110) according to any one of claims 1 to 3, wherein the second sealing lip (126) comprises at least one first portion (134) and at least one second portion (136).

5. The jacket (110) according to claim 4, wherein the first portion (134) at least partially runs along an outer edge (138) of the flexible envelope (114).

6. The jacket (110) according to any one of claims 4 or 5, wherein first portion ends (146) of the first portion (134) respectively contact second portion ends (144) of the second portion (136), wherein the first portion ends (146) of the first portion (134) are located on opposing sides relative to a longitudinal axis (148) of the flexible envelope (114).

7. The jacket (110) according to any one of claims 4 to 6, wherein the first portion (134) is a skin contacting portion (178), wherein the second portion (136) is an interlocking portion (180) configured to contact the opposing second side (120) of the flexible envelope (114) and configured to be arranged next to the first portion (134) thereby establishing a form-fit connection.

8. The jacket (110) according to any one of claims 1 to 7, wherein the envelope contacting sealing end (150) is made of a material which is more elastic than a material of a remaining portion of the flexible envelope (114).

9. The jacket (110) according to any one of claims 1 to 8, wherein one or both of the first sealing lip (124) and the second sealing lip (126) are one or both of inflatable sealing lips, compressible sealing lips.

10. The jacket (110) according to any one of claims 1 to 9, wherein the first side (116) at least partially comprises at least one adhesive material.

11. The jacket (110) according to any one of claims 1 to 10, wherein the flexible envelope (114) is at least partially made of at least one transparent material.

12. The jacket (110) according to any one of claims 1 to 11, wherein the first side (116) is at least partially colored with at least one first color, wherein the opposing second side (120) is at least partially colored with at least one second color, wherein the first color is different from the second color.

13. The jacket (110) according to any one of claims 1 to 12, wherein the jacket (110) additionally comprises at least one humidity sensor.

14. A method for watertight sealing a medical device, the method comprising the following steps:
a) mounting at least one medical device (110) on a skin site (168) of an extremity (164) of a user;
b) placing the jacket (110) according to any one of claims 1 to 13 onto the skin site (168) such that the first sealing lip (124) and the second sealing lip (126) surround the medical device (112); and
c) wrapping the flexible envelope (114) around the extremity (164) and fixedly connecting the envelope contacting sealing end (150) to the opposing second side (120) of the of flexible envelope (114).

## Patentansprüche

1. Mantel (110) zum wasserdichten Abdichten einer medizinischen Vorrichtung (112), wobei der Mantel (110) mindestens eine flexible Umhüllung (114) umfasst, wobei die flexible Umhüllung (114) mindestens eine erste Seite (116, 117), die der Haut eines Patienten zugewandt ist, und mindestens eine gegenüberliegende zweite Seite (120) umfasst, wobei die erste Seite (116) Folgendes umfasst:
• mindestens eine erste Dichtlippe (124), wobei die erste Dichtlippe (124) so ausgebildet ist, dass sie die medizinische Vorrichtung (112) umschließt; und
• mindestens eine zweite Dichtlippe (126), wobei die zweite Dichtlippe (126) so ausgebildet ist, dass sie die medizinische Vorrichtung (112) umschließt, wobei die zweite Dichtlippe (126) von der ersten Dichtlippe (124) beabstandet ist;
wobei die flexible Umhüllung (114) ferner mindestens ein die Umhüllung kontaktierendes Dichtende (150) umfasst, wobei die der Haut zugewandte erste Seite (117) des die Umhüllung kontaktierenden Dichtendes (150) fest mit der gegenüberliegenden zweiten Seite (120) der flexiblen Umhüllung (114) verbunden werden kann, wenn die flexible Umhüllung um eine Extremität eines Benutzers gewickelt wird,
wobei die zweite Dichtlippe (126) die erste Dichtlippe (124) umschließt.

2. Mantel (110) nach Anspruch 1, wobei die flexible Umhüllung (114) mindestens einen Bereich mit geringer Elastizität umfasst.

3. Mantel (110) nach einem der Ansprüche 1 oder 2, wobei die medizinische Vorrichtung (112) eine Höhe h_{md} der medizinischen Vorrichtung hat, wobei in einem komprimierten Zustand der ersten Dichtlippe (124) die erste Dichtlippe (124) eine Höhe der ersten Dichtlippe hat, wobei die Höhe der ersten Dichtlippe größer ist als die Höhe h_{md} der medizinischen Vorrichtung.

4. Mantel (110) nach einem der Ansprüche 1 bis 3, wobei die zweite Dichtlippe (126) mindestens einen ersten Abschnitt (134) und mindestens einen zweiten Abschnitt (136) umfasst.

5. Mantel (110) nach Anspruch 4, wobei der erste Abschnitt (134) mindestens teilweise entlang eines äußeren Randes (138) der flexiblen Umhüllung (114) verläuft.

6. Mantel (110) nach einem der Ansprüche 4 oder 5, wobei erste Abschnittsenden (146) des ersten Abschnitts (134) jeweils zweite Abschnittsenden (144) des zweiten Abschnitts (136) kontaktieren, wobei sich die ersten Abschnittsenden (146) des ersten Abschnitts (134) auf gegenüberliegenden Seiten in Bezug auf eine Längsachse (148) der flexiblen Umhüllung (114) befinden.

7. Mantel (110) nach einem der Ansprüche 4 bis 6, wobei der erste Abschnitt (134) ein die Haut kontaktierender Abschnitt (178) ist, wobei der zweite Abschnitt (136) ein Verschlussabschnitt (180) ist, der so ausgebildet ist, dass er die gegenüberliegende zweite Seite (120) der flexiblen Umhüllung (114) kontaktiert und neben dem ersten Abschnitt (134) angeordnet ist, wodurch eine formschlüssige Verbindung hergestellt wird.

8. Mantel (110) nach einem der Ansprüche 1 bis 7, wobei das die Umhüllung kontaktierende Dichtende (150) aus einem Material gefertigt ist, das elastischer ist als ein Material eines verbleibenden Abschnitts der flexiblen Umhüllung (114).

9. Mantel (110) nach einem der Ansprüche 1 bis 8, wobei eine oder beide von der ersten Dichtlippe (124) und der zweiten Dichtlippe (126) eines oder beides von aufblasbaren Dichtlippen, komprimierbaren Dichtlippen ist/sind.

10. Mantel (110) nach einem der Ansprüche 1 bis 9, wobei die erste Seite (116) mindestens teilweise mindestens ein Haftmaterial umfasst.

11. Mantel (110) nach einem der Ansprüche 1 bis 10, wobei die flexible Umhüllung (114) mindestens teilweise aus mindestens einem transparenten Material gefertigt ist.

12. Mantel (110) nach einem der Ansprüche 1 bis 11, wobei die erste Seite (116) mindestens teilweise mit mindestens einer ersten Farbe gefärbt ist, wobei die gegenüberliegende zweite Seite (120) mindestens teilweise mit mindestens einer zweiten Farbe gefärbt ist, wobei die erste Farbe verschieden ist von der zweiten Farbe.

13. Mantel (110) nach einem der Ansprüche 1 bis 12, wobei der Mantel (110) zusätzlich mindestens einen Feuchtigkeitssensor umfasst.

14. Verfahren zum wasserdichten Abdichten einer medizinischen Vorrichtung, wobei das Verfahren die folgenden Schritte umfasst:
a) Befestigen mindestens einer medizinischen Vorrichtung (110) an einer Hautstelle (168) einer Extremität (164) eines Benutzers;
b) Platzieren des Mantels (110) nach einem der Ansprüche 1 bis 13 auf der Hautstelle (168) derart, dass die erste Dichtlippe (124) und die zweite Dichtlippe (126) die medizinische Vorrichtung (112) umschließen; und
c) Wickeln der flexiblen Umhüllung (114) um die Extremität (164) und festes Verbinden des die Umhüllung kontaktierenden Dichtendes (150) mit der gegenüberliegenden zweiten Seite (120) der flexiblen Umhüllung (114).

## Revendications

1. Gaine (110) pour le scellement étanche d'un dispositif médical (112), dans laquelle la gaine (110) comprend au moins une enveloppe souple (114), dans laquelle l'enveloppe souple (114) comprend au moins un premier côté (116, 117) pour faire face à la peau d'un patient et au moins un second côté (120) opposé, dans laquelle le premier côté (116) comprend :
• au moins une première lèvre de scellement (124), la première lèvre de scellement (124) étant conçue pour entourer le dispositif médical (112) ; et
• au moins une seconde lèvre de scellement (126), la seconde lèvre de scellement (126) étant conçue pour entourer le dispositif médical (112), la seconde lèvre de scellement (126) étant espacée de la première lèvre de scellement (124) ;
dans laquelle l'enveloppe souple (114) comprend en outre au moins une extrémité de scellement (150) en contact avec l'enveloppe, dans laquelle le premier côté (117) faisant face à la peau de l'extrémité de scellement (150) en contact avec l'enveloppe peut être relié de manière fixe au second côté (120) opposé de l'enveloppe souple (114) lors de l'enroulement de l'enveloppe souple autour d'une extrémité d'un utilisateur,
dans laquelle la seconde lèvre de scellement (126) entoure la première lèvre de scellement (124).

2. Gaine (110) selon la revendication 1, dans laquelle l'enveloppe souple (114) comprend au moins une section de faible élasticité.

3. Gaine (110) selon l'une quelconque des revendications 1 à 2 dans laquelle le dispositif médical (112) a une hauteur de dispositif médical h_{dm}, dans laquelle, dans un état comprimé de la première lèvre de scellement (124), la première lèvre de scellement (124) a une hauteur de première lèvre de scellement, dans laquelle la hauteur de la première lèvre de scellement est supérieure à la hauteur du dispositif médical h_{dm}.

4. Gaine (110) selon l'une quelconque des revendications 1 à 3, dans laquelle la seconde lèvre de scellement (126) comprend au moins une première partie (134) et au moins une seconde partie (136).

5. Gaine (110) selon la revendication 4, dans laquelle la première partie (134) longe au moins partiellement un bord externe (138) de l'enveloppe souple (114).

6. Gaine (110) selon l'une quelconque des revendications 4 ou 5, dans laquelle les extrémités de première partie (146) de la première partie (134) entrent respectivement en contact avec les extrémités de seconde partie (144) de la seconde partie (136), dans laquelle les extrémités de première partie (146) de la première partie (134) sont situées sur des côtés opposés par rapport à un axe longitudinal (148) de l'enveloppe souple (114).

7. Gaine (110) selon l'une quelconque des revendications 4 à 6, dans laquelle la première partie (134) est une partie en contact avec la peau (178), dans laquelle la seconde partie (136) est une partie d'emboîtement (180) conçue pour entrer en contact avec le second côté (120) opposé de l'enveloppe souple (114) et conçue pour être agencée à côté de la première partie (134), établissant ainsi une liaison à correspondance de forme.

8. Gaine (110) selon l'une quelconque des revendications 1 à 7, dans laquelle l'extrémité de scellement (150) en contact avec l'enveloppe est constituée d'un matériau qui est plus élastique qu'un matériau d'une partie restante de l'enveloppe souple (114).

9. Gaine (110) selon l'une quelconque des revendications 1 à 8, dans laquelle une ou les deux parmi la première lèvre de scellement (124) et la seconde lèvre de scellement (126) sont une ou les deux parmi des lèvres de scellement gonflables, des lèvres de scellement compressibles.

10. Gaine (110) selon l'une quelconque des revendications 1 à 9, dans laquelle le premier côté (116) comprend au moins partiellement au moins un matériau adhésif.

11. Gaine (110) selon l'une quelconque des revendications 1 à 10, dans laquelle l'enveloppe souple (114) est au moins partiellement constituée d'au moins un matériau transparent.

12. Gaine (110) selon l'une quelconque des revendications 1 à 11, dans laquelle le premier côté (116) est au moins partiellement coloré avec au moins une première couleur, dans laquelle le second côté (120) opposé est au moins partiellement coloré avec au moins une deuxième couleur, dans laquelle la première couleur est différente de la deuxième couleur.

13. Gaine (110) selon l'une quelconque des revendications 1 à 12, dans laquelle la gaine (110) comprend de plus au moins un capteur d'humidité.

14. Procédé de scellement étanche d'un dispositif médical, le procédé comprenant les étapes suivantes :
a) montage d'au moins un dispositif médical (110) sur un site de peau (168) d'une extrémité (164) d'un utilisateur ;
b) placement de la gaine (110) selon l'une quelconque des revendications 1 à 13 sur le site de peau (168) de telle sorte que la première lèvre de scellement (124) et la seconde lèvre de scellement (126) entourent le dispositif médical (112) ; et
c) enroulement de l'enveloppe souple (114) autour de l'extrémité (164) et liaison fixe de l'extrémité de scellement (150) en contact avec l'enveloppe au second côté (120) opposé de l'enveloppe souple (114).
